# EUROPÄISCHE PATENTSCHRIFT

(11) **EP 2 101 860 B1**
(45) Veröffentlichungstag und Bekanntmachung des Hinweises auf die Patenterteilung: **05.08.2015**
(21) Anmeldenummer: 07847029.1
(22) Anmeldetag: 07.12.2007
(51) Int. Cl.: A61M 39/28, F16K 7/02

(54) **QUETSCHVENTIL**
PINCH VALVE
CLAPET À STRICTION

(30) Priorität: 18.01.2007 DE 102007002765
(43) Veröffentlichungstag der Anmeldung: 23.09.2009
(73) Patentinhaber: Festo AG & Co. KG, 73734 Esslingen (DE)
(72) Erfinder: KEES, Ulrich, 66424 Homburg (DE); RÖHRIG, Harald, 66583 Spiesen-Elversberg (DE)
(74) Vertreter: Patentanwälte Magenbauer & Kollegen Partnerschaft mbB
(86) Internationale Anmeldenummer: PCT/EP2007/010682
(87) Internationale Veröffentlichungsnummer: WO 2008/086849

(56) Entgegenhaltungen:
- DE-C1- 19 900 320
- US-A- 5 445 613
- US-B1- 6 279 869
- US-B1- 6 637 723

## Beschreibung

Die Erfindung betrifft ein Quetschventil, mit einem sich zwischen zwei Anschlussstücken erstreckenden, eine flexible Umfangswand aufweisenden schlauchförmigen Ventilglied, im Bereich dessen Außenumfanges mindestens ein zum mehr oder weniger starken Zusammenquetschen der Umfangswand unter Ausführung einer Arbeitsbewegung quer zu der Ventilglied-Längsachse bewegbares Quetschelement angeordnet ist, mit im Bereich des Außenumfanges des Ventilgliedes angeordneten Beaufschlagungsmitteln, die zu einer Beaufschlagungsbewegung antreibbar sind, um durch Zusammenwirken mit dem mindestens einen Quetschelement dessen Arbeitsbewegung hervorzurufen, mit einem Ventilgehäuse mit einer umfangsseitigen Ventilgehäusewand, die einen die Beaufschlagungsmittel enthaltenden Arbeitsraum begrenzt, und mit für elektrische Signalabgabe ausgelegten Positionserfassungsmitteln zur berührungslosen Erfassung mindestens einer im Rahmen der Beaufschlagungsbewegung einnehmbaren Betriebsposition der Beaufschlagungsmittel, wobei die Positionserfassungsmittel die Beaufschlagungsbewegung mitmachende Betätigungsmittel sowie außerhalb des Arbeitsraumes ventilgehäusefest angeordnete, berührungslos auf die Betätigungsmittel ansprechende Sensormittel enthalten.

Ein aus der DE 29 41 278 C2 bekanntes Quetschventil ist als Kanülenanschlussstück ausgebildet, das in der Medizintechnik eingesetzt wird. Es enthält ein von einem Fluid durchströmbares schlauchförmiges Ventilglied, an dessen Außenumfang zwei radial bewegliche Quetschelemente angeordnet sind, die von einem klammerartigen Beaufschlagungsglied umgriffen werden. Das Beaufschlagungsglied ist als manuell betätigbarer Schieber ausgeführt, der mit schrägen Beaufschlagungsflächen an den Quetschelementen angreift und bei dessen Betätigung die Quetschelemente so aufeinander zu gerichtet verlagerbar sind, dass das Ventilglied lokal zusammengequetscht und der Fluiddurchfluss dadurch verringert oder komplett unterbunden wird.

Ein aus der DE 22 43 173 A hervorgehendes Quetschventil verfügt in einer in Figuren 10 und 11 der DE 22 23 173 A abgebildeten Ausführungsform über ein schlauchförmiges Ventilglied, das von Quetschmitteln in Gestalt eines von Kugeln umgebenen flexiblen Ringes umschlossen ist. Ebenfalls koaxial zu dem Ventilglied angeordnete Beaufschlagungsmittel in Gestalt eines Beaufschlagungsringes sind durch Fluidbeaufschlagung längs des Ventilgliedes bewegbar, um auf die Quetschmittel einzuwirken und diese quer dazu zu verlagern, um den Querschnitt des Ventilgliedes zu verändern.

Eine ähnliche Anordnung offenbart die DE 75 0 720 U. Bei einer möglichen Ausführungsform sind dort die Beaufschlagungsmittel als Schiebehülse ausgebildet, während als Ventilglied ein in eine Bohrung eingesteckter Schlauch zum Einsatz kommt. Die Schiebehülse ist für manuelle Betätigung ausgelegt.

Bei dem Quetschventil der DE 33 18 817 A1 kann zur Betätigung über eine verdrehbare und verschiebbare Nockenstange auf ein Druckstück eingewirkt werden, sodass das Ventilglied mehr oder weniger stark zusammengequetscht wird.

Bei diesen bekannten Quetschventilen kann die momentan getroffene Durchflusseinstellung visuell anhand der von außen sichtbaren Betriebsposition der Beaufschlagungsmittel festgestellt werden. Allerdings ist diese Visualisierung relativ ungenau.

Aus der Produktinformation "Mechanische Quetschventile Typ BSV / Mechanical pinch valves type BSV" der AKO Armaturen & Separations GmbH, D-65468 Trebur-Astheim, Ausgabe 10/2001, sind pneumatische Quetschventile bekannt, bei denen die zum Schließen erforderliche Kraft durch Federmittel aufgebracht wird und das Öffnen mittels Druckluft erfolgt. Ein zur Betätigung dienender Pneumatikzylinder ist seitlich an das Ventilgehäuse angebaut, was insgesamt eine sehr voluminöse Bauweise zur Folge hat. Als Zubehörteile sind Endlageninitiatoren erwähnt, mit denen sich vermutlich die Endlagen des Pneumatikzylinders erfassen lassen, wie dies bei Pneumatikzylindern, unabhängig von ihrem Einsatz, allgemein üblich ist.

Die DE 199 00 320 C1 zeigt ein nach dem Prinzip eines Quetschventils arbeitendes Sicherheitsventil mit einem von einer Schlauchleitung gebildeten Ventilglied und an dessen Außenumfang angeordneten, quer zur Längsachse des Ventilgliedes bewegbaren Quetschelementen. Mittels außen am Gehäuse des Sicherheitsventils angeordneten Positionssensoren lässt sich die quer zum Ventilglied eingenommene Stellung der Quetschelemente erfassen. Auch dieses Quetschventil weist quer zur Längsachse des Ventilgliedes relativ große Abmessungen auf.

Die US 5 445 613 offenbart ein Quetschventil der eingangs genannten Art, das ein auf einen Schlauch einwirkendes Quetschelement aufweist, das durch Beaufschlagungsmittel in Gestalt einer Stange und eines Kolbens antreibbar ist. An dem Quetschelement ist ein Magnet angeordnet, der mit einem Sensor zusammenwirkt, um die Betätigung der Beaufschlagungsmittel zu steuern.

Aus der US 6 637 723 ist ein Fluidventil bekannt, das eine von einem Fluidkanal durchsetzte Stange aufweist, die von einem Ventilglied nach Art einer Manschette umschlossen ist. Zur Betätigung des Ventilgliedes dient ein die Stange axial verschiebbar umschließendes Betätigungselement.

Eine wesentliche Aufgabe der vorliegenden Erfindung besteht darin, ein kompakt bauendes Quetschventil zu schaffen, das eine zuverlässige Betriebsüberwachung ermöglicht.

Zur Lösung dieser Aufgabe ist vorgesehen, dass die Ventilgehäusewand das Ventilglied radial außen umschließt und dass die Beaufschlagungsbewegung der Beaufschlagungsmittel längs des Ventilgliedes orientiert ist, wobei die Betätigungsmittel an den Beaufschlagungsmitteln angeordnet sind.

Somit sind die Beaufschlagungsmittel in einem Ventilgehäuse untergebracht, was eine Abschirmung zur Umgebung hin bewirkt und mithin eine Verringerung der Verletzungs- und Verschmutzungsgefahr. Die hierbei beibehaltene Parallelausrichtung der Beaufschlagungsbewegung und der Ventilglied-Längsachse gewährleistet kompakte Außenabmessungen. Die zusätzlichen Positionserfassungsmittel ermöglichen auf der Basis der Ausgabe eines oder mehrerer elektrischer Signale trotz von außen nicht sichtbarer Beaufschlagungsmittel eine zuverlässige Betriebsüberwachung und somit auch eine problemlose Integration in elektronische Ansteuerungsmaßnahmen. Die Positionserfassungsmittel enthalten an den Beaufschlagungsmitteln angeordnete Betätigungsmittel, die die Bewegung der Beaufschlagungsmittel mitmachen und die mit ventilgehäusefesten Sensormitteln kooperieren, die außerhalb des Arbeitsraumes am Ventilgehäuse angebracht sind.

Vorteilhafte Weiterbildungen der Erfindung gehen aus den Unteransprüchen hervor.

Besonders einfach und zuverlässig sind Positionserfassungsmittel, bei denen die Betätigungsmittel aus einer Permanentmagneteinrichtung bestehen, die beispielsweise mindestens einen das Ventilglied koaxial umschließenden Ringmagnet enthalten kann.

Die Sensormittel können sowohl ausgebildet sein, um nur bestimmte Positionen zu erfassen, als auch zur kontinuierlichen Positionserfassung, also als sogenanntes Wegmesssystem.

Um eine optimale Justierung zu ermöglichen, sind die Sensormittel zweckmäßigerweise in Richtung der Beaufschlagungsbewegung einstellbar am Ventilgehäuse angeordnet.

Zur Fixierung der Sensormittel ist die umfangsseitige Ventilgehäusewand zweckmäßigerweise mit mindestens einer sich in der Richtung der Beaufschlagungsbewegung erstreckenden Sensoraufnahme versehen. Hierbei handelt es sich beispielsweise um eine längsseits offene, hinterschnittene Längsnut oder aber um einen bohrungsartigen Kanal. Letzterer kann bei Bedarf auch dazu verwendet werden, um stirnseitig Befestigungsschrauben einzuschrauben, mit denen stirnseitige Abschlusselemente an die umfangsseitige Ventilgehäusewand anbaubar sind.

Die mindestens eine Sensoraufnahme kann insbesondere in einer rippenartigen Erhebung am Außenumfang der umfangsseitigen Ventilgehäusewand ausgebildet sein. Auf diese Weise kann trotz im Übrigen geringer Wandstärke dieser Ventilgehäusewand ausreichend Material zur sicheren Fixierung der Sensormittel zur Verfügung gestellt werden.

Die umfangsseitige Ventilgehäusewand ist bevorzugt von einem unter Bildung der mindestens einen Sensoraufnahme hergestellten Profilrohr gebildet. Dieses kann auf der Basis eines Strangspressteils in praktisch beliebiger Länge hergestellt werden.

Eine besonders zuverlässige Funktion ist gewährleistet, wenn das Quetschventil über zwei Quetschelemente verfügt, die sich am Außenumfang des Ventilgliedes diametral gegenüberliegen und die gegensinnig bewegbar sind.

Enthalten die Beaufschlagungsmittel als mit dem mindestens einen Quetschelement zusammenwirkende Komponente einen das Ventilglied koaxial umschließenden Beaufschlagungsring, kann eine symmetrische Krafteinleitung erfolgen, was vor allem bei hohen Stellkräften von Vorteil ist. Verkantungen können somit wirksam vermieden werden.

Die den Beaufschlagungsmitteln zugeordneten Betätigungsmittel der Positionserfassungsmittel sind zweckmäßigerweise an dem vorgenannten Beaufschlagungsring angeordnet. Auf diese Weise ist eine sehr genaue Rückmeldung der aktuellen Betriebsposition der Beaufschlagungsmittel möglich.

Geeignete Antriebsmittel sind zweckmäßigerweise in der Lage, zur Erzeugung einer die Beaufschlagungsbewegung hervorrufenden Antriebskraft beizutragen, ohne dass ein manueller Zugriff zu den Beaufschlagungsmitteln notwendig wäre. Prinzipiell wäre jeder Antriebsmechanismus denkbar, beispielsweise auch elektromagnetischer Art und/oder mittels Hebelübersetzung. Bevorzugt sind die Antriebsmittel jedoch ausgebildet, um die Antriebskraft fluidisch zu erzeugen. Hierbei wird eine pneumatische Bauart bevorzugt.

In Verbindung mit fluidischen Antriebsmitteln befindet sich in dem von der umfangsseitigen Ventilgehäusewand umgrenzten Arbeitsraum axial benachbart zu den Beaufschlagungsmitteln zweckmäßigerweise eine Antriebskammer, die gesteuert mit einem Antriebsfluid beaufschlagbar ist. Dieses Antriebsfluid wirkt dann auf die Beaufschlagungsmittel ein, um diese wunschgemäß zu positionieren.

Die Fluidbeaufschlagung erfolgt insbesondere über einen das Ventilgehäuse des Quetschventils zur Außenfläche hin durchsetzenden Steuerkanal. An diesen Steuerkanal kann beispielsweise eine zu einem externen Steuerventil führende fluidische Steuerleitung angeschlossen werden. Alternativ könnte auch unmittelbar an die Außenfläche des Ventilgehäuses ein Steuerventil angebaut sein, das den Fluiddurchfluss durch den Steuerkanal kontrolliert.

Als besonders zweckmäßig wird es angesehen, die Beaufschlagungsmittel in einen mit dem mindestens einen Quetschelement kooperierenden Beaufschlagungsring und einen diesbezüglich gesonderten, die Antriebskammer begrenzenden Antriebskolben zu unterteilen. Der Antriebskolben schirmt den Beaufschlagungsring von der Antriebskammer ab und verhindert somit einen Kontakt des Antriebsfluides mit dem Beaufschlagungsring und den diesem zugeordneten Quetschelementen. Hierbei ist es dann auch besonders vorteilhaft, die Betätigungsmittel der Positionserfassungsmittel außerhalb der Antriebskammer und insbesondere an dem Beaufschlagungsring anzubringen. Die Betätigungsmittel sind dadurch wirksam vor Verunreinigungen geschützt, die das Antriebsfluid möglicherweise mit sich führt.

Der Antriebskolben kann zwar fest mit dem Beaufschlagungsring verbunden sein und mit diesem eine Baueinheit bilden. Bevorzugt wird jedoch eine Bauform, bei der diese Komponenten getrennt voneinander ausgebildet sind. In diesem Fall kann der Antriebskolben üblicherweise nur Druckkräfte, jedoch keine Zugkräfte auf den Beaufschlagungsring ausüben. Die damit verbundene Entkopplung ermöglicht quer zur Ventilglied-Längsachse voneinander unabhängige Relativbewegungen zwischen den beiden Komponenten, sodass Reaktionskräfte, die aus der Kraftumlenkung in die Quetschelemente resultieren, vom Antriebskolben ferngehalten werden und dessen Dichtungen und Gleitflächen keinem erhöhten Verschleiß unterliegen.

Eine ebenfalls in der Arbeitskammer untergebrachte Federeinrichtung kann eine der fluidischen Antriebskraft entgegenwirkende Feder-Rückstellkraft liefern. Die Federeinrichtung ist vorzugsweise koaxial bezüglich des Ventilgliedes angeordnet, zweckmäßigerweise in axialer Verlängerung des gegebenenfalls vorhandenen Beaufschlagungsringes. Sie befindet sich vorzugsweise auf der dem Antriebsraum axial entgegengesetzten Seite der Arbeitskammer.

Durch die Federeinrichtung kann eine Beaufschlagung des mindestens einen Quetschelementes in eine Grundstellung bewirkt werden. Bei dieser Grundstellung handelt es sich zweckmäßigerweise um eine Schließstellung, bei der die Quetschelemente die Umfangswand des Ventilgliedes so weit zusammengequetscht haben, dass der Fluiddurchgang durch den das Ventilglied durchsetzenden Fluidkanal unterbrochen ist. Bei Bedarf kann aber auch eine umgekehrte Auslegung vorhanden sein, sodass die Federeinrichtung entlastend wirkt und somit die Quetschelemente bei nicht vorhandener Fluidbeaufschlagung der Beaufschlagungsmittel eine Offenstellung einnehmen können, in der sie einen ungehinderten Fluiddurchgang durch das schlauchförmige Ventilglied zulassen.

Um zu verhindern, dass das schlauchförmige Ventilglied von dem in ihm herrschenden Fluiddruck unkontrolliert aufgebläht wird, ist es zweckmäßigerweise zumindest im Bereich seiner flexiblen Umfangswand von einer hülsenartigen Abstützeinrichtung koaxial umschlossen. Der Innenquerschnitt der Abstützeinrichtung gibt mithin den maximalen Durchflussquerschnitt des Ventilgliedes vor.

In bevorzugter Ausgestaltung enthält die Abstützeinrichtung zwei Abstützhülsen, deren einander zugewandte Stirnflächen einen Führungsspalt definieren, in dem das mindestens eine Quetschelement zur Ausführung seiner Arbeitsbewegung radial verschiebbar geführt ist. Die Führungsmaßnahmen sind insbesondere so ausgeführt, dass eine Linearführung für die Quetschelemente vorhanden ist, die gleichzeitig rechtwinkelig zur Arbeitsbewegung und zur Ventilglied-Längsachse eine Abstützung der Quetschelemente bewirkt, sodass diese in ihrer Relativposition bezüglich des Ventilgliedes stabilisiert sind und auch keine Verkantungen auftreten können.

Die den Arbeitsraum umfangsseitig begrenzende Ventilgehäusewand hat zweckmäßigerweise zumindest größtenteils eine kreiszylindrische Außenkontur.

Bei dem schlauchförmigen Ventilglied handelt es sich zweckmäßigerweise um ein gummielastisches Formteil mit einer im unbelasteten Ausgangszustand von einer rein kreiszylindrischen Gestaltung abweichenden Formgebung. Bevorzugt ist die Umfangswand so gestaltet, dass sie allein aufgrund ihrer eigenen Formstabilität eine sich von axial außen her in Richtung zum Angriffsbereich der Quetschelemente hin verjüngende Formgebung aufweist. Die Umfangswand kann in den mit den Quetschelementen kooperierenden Umfangsabschnitten abgeflacht sein, was insbesondere dann von Vorteil ist, wenn der das Ventilglied kontaktierende Quetschabschnitt der Quetschelemente eine lineare Erstreckung quer zur Richtung der Arbeitsbewegung aufweist. Somit können übermäßige Verformungen der Umfangswand des Ventilgliedes vermieden werden.

Vorzugsweise handelt es sich bei dem schlauchförmigen Ventilglied insgesamt um ein einstückiges, aus Material mit gummielastischen Eigenschaften bestehendes Formteil. Als Material kommt insbesondere ein Elastomermaterial in Frage.

Nachfolgend wird die Erfindung anhand der beiliegenden Zeichnung näher erläutert. In dieser zeigen:
- Fig. 1 und 2: perspektivische Außenansichten eines erfindungsgemäßen Quetschventils bevorzugten Aufbaus aus unterschiedlichen Blickrichtungen,
- Fig. 3: das Quetschventil aus Figuren 1 und 2 in der maximalen Offenstellung und in einem Längsschnitt gemäß Schnittlinie III-III aus Figur 5,
- Fig. 4: das Quetschventil in der Schließstellung und in einem Längsschnitt gemäß Schnittlinie IV-IV aus Figur 6,
- Fig. 5: einen Querschnitt gemäß Schnittlinie V-V durch das in Offenstellung befindliche Quetschventil,
- Fig. 6: einen Querschnitt gemäß Schnittlinie VI-VI durch das in Schließstellung befindliche Quetschventil,
- Fig. 7: eine Seitenansicht des Quetschventils mit Blickrichtung gemäß VII aus Figuren 3 und 4 und
- Fig. 8: eine Stirnansicht der umfangsseitigen Ventilgehäusewand einer modifizierten Bauform des Quetschventils.

Das in seiner Gesamtheit mit Bezugsziffer 1 bezeichnete Quetschventil enthält ein Ventilgehäuse 2, das in seinem Innern einen kreiszylindrisch konturierten Arbeitsraum 3 definiert.

Bestandteil des Ventilgehäuses 2 ist eine rohrförmige Ventilgehäusewand 5, die den Arbeitsraum 3 radial außen begrenzt und die im Folgenden als umfangsseitige Ventilgehäusewand 5 bezeichnet wird.

An den beiden Stirnseiten der umfangsseitigen Ventilgehäusewand 5 ist je eine stirnseitige Abschlusswand 6a, 6b angeordnet, durch die der Arbeitsraum 3 axial begrenzt ist. Diese Abschlusswände 6a, 6b können wie abgebildet ein Außengewinde aufweisen, mit dem sie stirnseitig in die umfangsseitige Ventilgehäusewand 5 eingeschraubt sind. Wenigstens eine der Abschlusswände 6a, 6b kann auch einstückig mit der umfangsseitigen Ventilgehäusewand 5 ausgeführt sind.

Im noch nicht montierten Zustand mindestens einer stirnseitigen Abschlusswand 6 ist der Innenraum der umfangsseitigen Ventilgehäusewand 5 stirnseitig frei zugänglich und ermöglicht so die Montage oder Demontage der weiteren Bestandteile des Quetschventils 1.

Im Bereich der beiden axialen Endabschnitte des Ventilgehäuses 2 befindet sich je ein Anschlussstück 15, 16, das axial von einem Fluidkanal 17 durchsetzt ist. Zweckmäßigerweise sind die Anschlussstücke 15, 16 unmittelbar von der jeweils zugeordneten stirnseitigen Abschlusswand 6a, 6b gebildet.

Zwischen den beiden Anschlussstücken 15, 16 erstreckt sich im Innern des Arbeitsraumes 3 mit koaxialer Ausrichtung ein mit einer flexiblen Umfangswand 24 ausgestattetes schlauchförmiges Ventilglied 25. Dieses steht mit den beiden Anschlussstücken 15, 16 in fluiddichter Verbindung und enthält einen von der Umfangswand 24 umschlossenen Durchgangskanal 26, der mit den Fluidkanälen 17 der beiden Anschlussstücke 15, 16 kommuniziert.

Über je eines der Anschlussstücke 15 oder 16 kann zu steuerndes fluidisches Druckmedium eingespeist werden, das durch den Durchgangskanal 26 zum jeweils anderen Anschlussstück gelangt und über dieses das Quetschventil 1 wieder verlässt. An den beiden Anschlussstücken 15, 16 angeordnete Befestigungsmittel 27; beispielsweise in die Fluidkanäle 17 eingebrachte Innengewinde, erlauben den Anschluss von Fluidleitungen, die das zu steuernde Fluid heranführen beziehungsweise abführen.

Im Bereich des Außenumfanges der Umfangswand 24 des Ventilgliedes 25 sind zwei Quetschelemente 28a, 28b angeordnet. Sie liegen in Richtung der Ventilglied-Längsachse 32 auf gleicher axialer Höhe, und zwar sich bezüglich der Ventilglied-Längsachse 32 diametral gegenüberliegend. Beide Quetschelemente 28a, 28b sind unabhängig voneinander quer und insbesondere rechtwinkelig zur Ventilglied-Längsachse 32 beweglich, wobei die entsprechenden Bewegungen als Arbeitsbewegungen 33 bezeichnet seien, die in der Zeichnung durch Doppelpfeile angedeutet sind. Die Arbeitsbewegungen 33 sind zweckmäßigerweise Linearbewegungen mit zusammenfallenden Achsrichtungen.

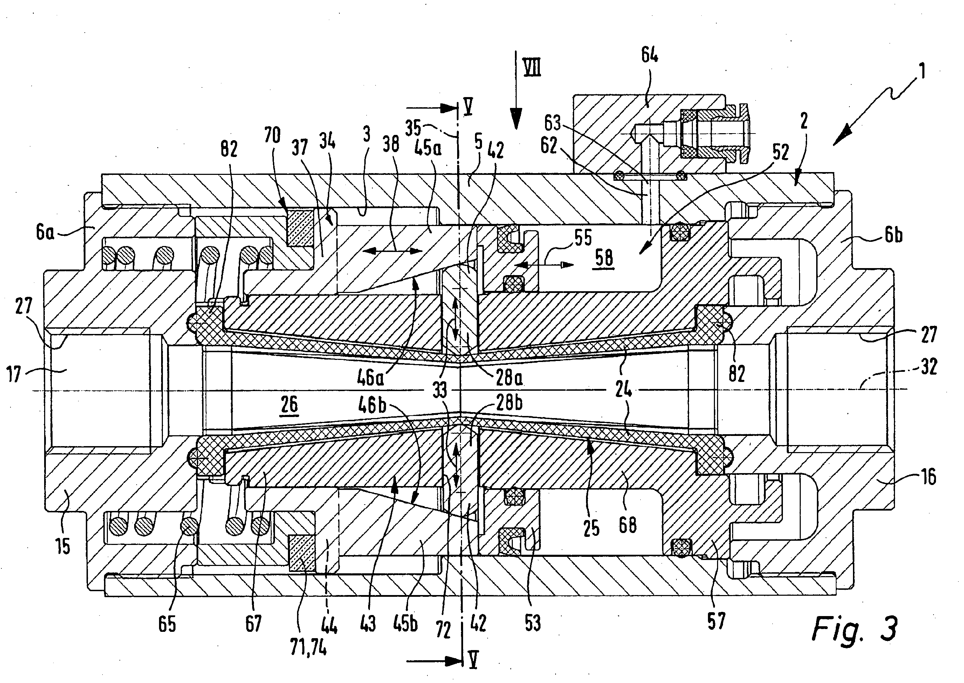

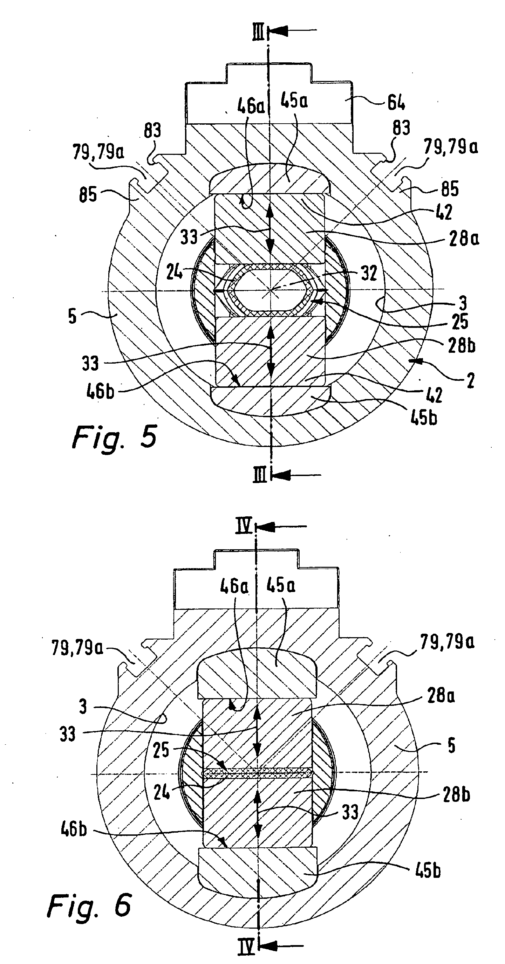

Wie man insbesondere den Figuren 3 bis 6 entnehmen kann, sind die Quetschelemente 28a, 28b bevorzugt plattenförmig gestaltet, wobei ihre Hauptausdehnungsebene rechtwinkelig zu der Ventilglied-Längsachse 32 verläuft. Bei Ausführung der Arbeitsbewegungen verlagern sie sich in einer gemeinsamen, mit den Hauptausdehnungsebenen zusammenfallenden Radialebene 35, die der Schnittebene in den Figuren 5 und 6 entspricht.

Die Quetschelemente 28a, 28b dienen zum mehr oder weniger starken Zusammenquetschen der Umfangswand 24. Ihre Arbeitsbewegungen finden stets gegensinnig statt, entweder aufeinander zu oder voneinander weg gerichtet.

In der in Figuren 3 und 5 gezeigten Offenstellung sind die beiden Quetschelemente 28a, 28b in Richtung der Arbeitsbewegungen 33 weitestmöglich voneinander entfernt, sodass die Umfangswand 24 keine oder eine nur geringe radiale Quetschung erfährt und der freigegebene Querschnitt des Durchgangskanals 26 ein Maximum aufweist.

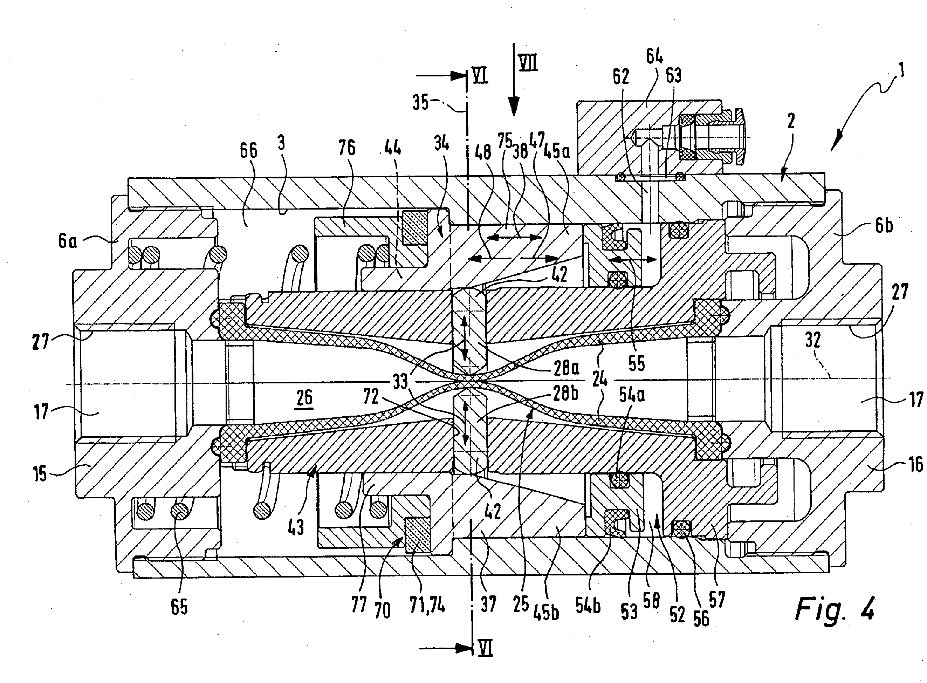

Unter Zusammenquetschen der Umfangswand 24 können die Quetschelemente 28a, 28b so weit aneinander angenähert werden, bis die aus Figuren 4 und 6 ersichtliche Schließstellung vorliegt, in der sie die Umfangswand 24 maximal zusammengequetscht haben und somit die sich in Richtung der Arbeitsbewegungen 33 gegenüberliegenden Wandabschnitte 36a, 36b der Umfangswand 24, die von den beiden Quetschelementen 28a, 28b beaufschlagt sind, fluiddicht zusammengepresst sind. Der Fluiddurchgang durch den Durchgangskanal 26 ist somit komplett abgesperrt. Die in der Offenstellung des Quetschventils 1 von den Quetschelementen 28a, 28b eingenommene Stellung maximalen gegenseitigen Abstandes sei im Folgenden als äußere Endstellung bezeichnet. Die maximal aneinander angenäherte Stellung der Quetschelemente 28a, 28b, die zur Vorgabe der Schließstellung des Quetschventils 1 vorliegt, sei als innere Endstellung bezeichnet.

Zwischenstellungen der Quetschelemente 28a, 28b zwischen der inneren und der äußeren Endstellung ermöglichen die stufenlose Vorgabe von unterschiedlichen Strömungsquerschnitten, die zwischen der Offenstellung und der Schließstellung des Ventilgliedes 25 liegen.

Abweichend vom Ausführungsbeispiel könnte auch nur ein einziges Quetschelement vorhanden sein. An die Stelle des anderen Quetschelementes tritt dann ein ventilgehäusefester Abstützabschnitt. In der Schließstellung ist dann die flexible Umfangswand 24 zwischen dem Abstützabschnitt und dem in die innere Endstellung bewegten Quetschelement zusammengequetscht. Den Quetschelementen 28a, 28b wird die zum Ausführen der Arbeitsbewegungen 33 erforderliche Stellkraft durch in ihrer Gesamtheit mit Bezugsziffer 34 bezeichnete Beaufschlagungsmittel auferlegt. Diese sind im Innern des Arbeitsraumes 3 angeordnet und dort in Richtung der Ventilglied-Längsachse 32 unter Ausführung einer durch einen Doppelpfeil angedeuteten Beaufschlagungsbewegung 38 relativ zum Ventilgehäuse 2 und zum Ventilglied 25 bewegbar. Bei dieser Beaufschlagungsbewegung 38 wirken die Beaufschlagungsmittel 34 mit den vom Ventilglied 25 wegweisenden äußeren Endabschnitten 42 der Quetschelemente 28a, 28b zusammen.

Die Beaufschlagungsmittel 34 sind insgesamt hülsen- oder ringförmig gestaltet und umschließen das schlauchförmige Ventilglied 25 koaxial, wobei sie ihrerseits von der umfangsseitigen Ventilgehäusewand 5 koaxial umschlossen sind. Auf diese Weise ergibt sich eine insgesamt äußerst kompakte Bauweise mit geringem Durchmesser des Quetschventils 1.

Zweckmäßigerweise befindet sich koaxial zwischen den Beaufschlagungsmittel 34 und dem schlauchförmigen Ventilglied 25 noch eine das Ventilglied 25 koaxial umschließende hülsenartige Abstützeinrichtung 43. Letztere erstreckt sich zwischen den beiden axialen Endabschnitten des Ventilgehäuses 2 und hat vor allem den Zweck, das Ventilglied 25 zumindest im Bereich seiner flexiblen Umfangswand 24 radial abzustützen und an einem unerwünschten Aufblähen durch den im Durchgangskanal 26 anstehenden Fluiddruck zu hindern. Die Innenkontur der hülsenartigen Abstützeinrichtung 43 entspricht der Außenkontur der Umfangswand 24 des Ventilgliedes 25, die diese aufweisen soll, wenn sich die Quetschelemente 28a, 28b in ihrer äußeren Endstellung befinden.

Zweckmäßigerweise enthalten die Beaufschlagungsmittel 34 einen im Arbeitsraum 3 koaxial zwischen der umfangsseitigen Ventilgehäusewand 5 und der hülsenförmigen Abstützeinrichtung 43 angeordneten Beaufschlagungsring 37. Er ist mit seinem Innenumfang auf dem Außenumfang der Abstützeinrichtung 43 und/oder mit seinem Außenumfang am Innenumfang der umfangsseitigen Ventilgehäusewand 5 verschiebbar geführt. Als mit der Abstützeinrichtung 43 kooperierendes Führungselement fungiert dabei ein ringförmiger Grundkörper 44 des Beaufschlagungsringes 37, der axial zwischen einerseits den beiden Quetschelementen 28a, 28b und andererseits einem der axialen Endabschnitte des Ventilgehäuses 2 angeordnet ist. Von diesem Grundkörper 44 ragen an der den Quetschelementen 28a, 28 zugewandten Seite zwei sich bezüglich der Ventilglied-Längsachse 32 diametral gegenüberliegende Beaufschlagungsflügel 45a, 45b axial weg, die jeweils eines der Quetschelemente 28a, 28b radial außen axial übergreifen. An der der Ventilglied-Längsachse 32 zugewandten Innenseite ist jeder Beaufschlagungsflügel 45a, 45b mit einer sich in Richtung der Beaufschlagungsbewegung 38 erstreckenden, bezüglich der Ventilglied-Längsachse 32 jedoch geneigten Beaufschlagungsfläche 46a, 46b versehen, die mit der nach radial außen orientierten, bevorzugt zumindest partiell ballig gestalteten Außenfläche des äußeren Endabschnittes 42 des jeweils zugeordneten Quetschelementes 28a, 28b in Berührkontakt steht. Bevorzugt sind die Beaufschlagungsflächen 46a, 46b eben und insbesondere in Gestalt einer schiefen Ebene ausgebildet. Sie entfernen sich jeweils zum freien Ende des Beaufschlagungsflügels 45a, 45b hin zunehmend von der Ventilglied-Längsachse 32.

Die Beaufschlagungsbewegung 38 besitzt eine zu den Quetschelementen 28a, 28b hin orientierte Aktivierungsrichtung 47 und eine dieser entgegengesetzte Deaktivierungsrichtung 48. Bei Verlagerung in der Aktivierungsrichtung 47 werden die Quetschelemente 28a, 28b durch die schrägen Beaufschlagungsflächen 46a, 46b allmählich in Richtung ihrer inneren Endstellung verlagert. Bewegt sich der Beaufschlagungsring 37 in der Deaktivierungsrichtung 48, werden die Quetschelemente 28a, 28b durch die sich infolge des in dem Durchgangskanal 26 herrschenden Fluiddruckes aufweitende Umfangswand 24 in Richtung der äußeren Endstellung nach radial außen zurückgeschoben. Auf diese Weise kann durch axiale Positionierung des Beaufschlagungsringes 37 der vom Durchgangskanal 26 zur Verfügung gestellte Strömungsquerschnitt und somit die Durchflussrate des zu steuernden Fluides variabel vorgegeben werden.

Zum Aufbringen der für die Beaufschlagungsbewegung 38 verantwortlichen Antriebskraft sind geeignete Antriebsmittel 52 vorhanden. Diese sind zweckmäßigerweise zur Ausübung einer fluidischen Antriebskraft ausgebildet, wobei die Fluidkraft vorzugsweise von unter einem entsprechenden Druck stehender Druckluft geliefert wird. Ein anderes fluidisches Druckmedium kann jedoch ebenfalls verwendet werden.

Die Antriebsmittel 52 enthalten einen beim Ausführungsbeispiel einen Bestandteil der Beaufschlagungsmittel 34 bildenden Antriebskolben 53, der mit der Fluidkraft beaufschlagt wird. Zweckmäßigerweise ist der Antriebskolben 53 ein bezüglich des Beaufschlagungsringes 37 separates Bauteil, das jedoch mechanisch mit dem Beaufschlagungsring 37 kooperiert.

Der Antriebskolben 53 ist als Ringkolben ausgebildet, der das Ventilglied 25 und die dieses umschließende Abstützeinrichtung 43 auf der dem Beaufschlagungsring 37 entgegengesetzten Axialseite der beiden Quetschelemente 28a, 28b koaxial umschließt. Er ist zu einer durch einen Doppelpfeil angedeuteten Antriebsbewegung 55 in Richtung der Ventilglied-Längsachse 32 antreibbar.

Mit seiner den Quetschelementen 28a, 28b zugewandten Stirnfläche kann der Antriebskolben 53 drückend auf den Beaufschlagungsring 37 einwirken. Hierzu liegt der Antriebskolben 53 zweckmäßigerweise an den ihm zugewandten Stirnflächen der Beaufschlagungsflügel 45a, 45b an. Dies ist zweckmäßigerweise ein loser Kontakt ohne feste Verbindung, sodass lediglich Druckkräfte, jedoch keine Zugkräfte ausübbar sind. Auf diese Weise sind Antriebskolben 53 und Beaufschlagungsring 37 quer zur Ventilglied-Längsachse 32 in ihren Bewegungen entkoppelt, was einen verschleißarmen Betrieb garantiert.

Der Antriebskolben 53 begrenzt eine auf der dem Beaufschlagungsring 37 entgegengesetzten Axialseite liegende Antriebskammer 58, die von einem Teilraum des Arbeitsraumes 3 definiert ist. Sie ist ebenfalls ringförmig ausgebildet. Über einen in sie einmündenden fluidischen Steuerkanal 62 kann die Antriebskammer 58 gesteuert mit einem Antriebsfluid beaufschlagt werden, um die Antriebsbewegung 55 in die eine oder andere Richtung hervorzurufen und demzufolge auch die daraus resultierende Beaufschlagungsbewegung 38 des Beaufschlagungsringes 37.

Die dem Antriebskolben 53 axial entgegengesetzte Begrenzung der Antriebskammer 58 übernimmt ein Flanschabschnitt 57 der Abstützeinrichtung 43, der der zugeordneten stirnseitigen Abschlusswand 6b axial innen vorgelagert ist und der unter Zwischenschaltung ringförmiger Dichtungsmittel 56 dichtend an der Innenumfangsfläche der umfangsseitigen Ventilgehäusewand 5 anliegt.

Der Steuerkanal 62 durchsetzt das Ventilgehäuse und mündet mit einer äußeren Kanalmündung 63 zur Außenfläche des Ventilgehäuses 2 aus, insbesondere zur peripheren Außenfläche der umfangsseitigen Ventilgehäusewand 5. Dort sind Anschlussmittel 64 angeordnet, die das Anschließen einer zu einer nicht weiter gezeigten Steuerventileinrichtung führenden Fluidleitung ermöglichen. Die Steuerventileinrichtung kann alternativ auch direkt ein Bestandteil des Quetschventils 1 sein. Beispielsweise könnte die Steuerventileinrichtung anstelle der Anschlussmittel 64 an der Außenfläche der Ventilgehäusewand 5 installiert sein.

Über die Steuerventileinrichtung kann die Fluidbeaufschlagung der Antriebskammer 58 nach Bedarf gesteuert werden, entweder durch Druckaufbau oder Druckabbau.

Von dem Antriebskolben 53 getragene ringförmige Dichtungsmittel 54a, 54b stehen in gleitverschieblichem Dichtkontakt mit der Außenumfangsfläche der Abstützeinrichtung 43 und der Innenumfangsfläche der umfangsseitigen Ventilgehäusewand 5. Dadurch ist das Antriebsfluid an einem Übertritt in denjenigen Bereich des Arbeitsraumes 3 gehindert, in dem sich der Beaufschlagungsring 37 befindet.

Der Antriebskolben 53 und der Beaufschlagungsring 37 könnten auch als Baueinheit ausgebildet sein.

Der Antriebskolben 53 kann vorliegend nur eine in der Deaktivierungsrichtung 48 wirksame Antriebskraft auf den Beaufschlagungsring 37 ausüben. Für die entgegengesetzte Antriebskraft sorgt eine ebenfalls in dem Arbeitsraum 3 untergebrachte Federeinrichtung 65, bei der es sich zweckmäßigerweise um eine mechanische Federeinrichtung handelt, insbesondere um eine Druckfedereinrichtung. Die Federeinrichtung 65 ist in einer Federkammer 66 untergebracht, bei der es sich um denjenigen Teilraum des Arbeitsraumes 3 handelt, welcher auf der dem Antriebskolben 53 entgegengesetzten Axialseite des Beaufschlagungsringes 37 zwischen diesem und der gegenüberliegenden stirnseitigen Abschlusswand 6a angeordnet ist.

Exemplarisch umschließt die mechanische Federeinrichtung 65 das Ventilglied 25 und die zugeordnete Abstützeinrichtung 43 koaxial, wobei sie sich einenends am Beaufschlagungsring 37 und andernends an der gegenüberliegenden stirnseitigen Abschlusswand 6a abstützt.

Somit ist der Beaufschlagungsring 37 durch die Federeinrichtung 65 ständig in der Aktivierungsrichtung 47 und mithin im Sinne eines Zusammenquetschens des Ventilgliedes 25 beaufschlagt. Soll also das Ventilglied 25 in Richtung der Schließstellung verformt werden, kann dies durch ein Ablassen des Antriebsfluides aus der Antriebskammer 58 hervorgerufen werden, da dies ein Zurückweichen des Antriebskolbens 53 ermöglicht. Dies hat auch den Vorteil, dass das Ventilglied 25 automatisch in die Schließstellung gequetscht wird, wenn aufgrund einer Fehlfunktion der in der Antriebskammer 58 herrschende Fluiddruck abfallen sollte. Das Quetschventil besitzt hier also die Konfiguration "normalerweise geschlossen".

Wird die Anordnung des Antriebskolbens 53 und der mechanischen Federeinrichtung 65 vertauscht, lässt sich bei Bedarf auch eine Konfiguration "normalerweise geöffnet" realisieren.

Um beim Ausführungsbeispiel das Ventilglied 25 zu öffnen, wird das Arbeitsfluid derart in die Antriebskammer 28 eingespeist, dass sich der Antriebskolben 53 in der Deaktivierungsrichtung 48 verlagert und durch ihn der Beaufschlagungsring 37 entgegen der durch die Federeinrichtung 65 aufgebrachten Federkraft zurückgeschoben wird.

Die Abstützeinrichtung 43 umfasst beim Ausführungsbeispiel zwei unter koaxialer Ausrichtung axial aufeinanderfolgende Abstützhülsen 67, 68, die unter Bildung eines die Quetschelemente 28a, 28b aufnehmenden Führungsspaltes 72 axial zueinander beabstandet sind. In dem Führungsspalt 72 sind die Quetschelemente 28a, 28b in Richtung ihrer Arbeitsbewegungen 33 verschiebbar geführt. An der einen Abstützhülse 68 ist, bevorzugt einstückig, der oben erwähnte Flanschabschnitt 57 angeordnet.

Bei dem Ventilglied 25 handelt es sich vorzugsweise um ein zumindest im Bereich seiner Umfangswand 24 und vorzugsweise insgesamt gummielastisches Formteil. Zweckmäßigerweise handelt es sich um einen einstückigen, aus Material mit gummielastischen Eigenschaften bestehenden Körper aus Gummi oder Elastomermaterial.

Zur Fixierung im Quetschventil 1 ist das Ventilglied 25 im Bereich seiner beiden Endabschnitte zweckmäßigerweise mit je einem radial abstehenden Befestigungsflansch 82 versehen, der axial zwischen der benachbarten stirnseitigen Abstützwand 6a, 6b und der dieser gegenüberliegenden Abstützhülse 67, 68 eingespannt ist. Da auch der Befestigungsflansch 82 aus Material mit gummielastischen Eigenschaften besteht, ergibt sich hierdurch eine fluiddichte Verbindung zu dem zugeordneten Anschlussstück 15, 16.

Vorzugsweise handelt es sich bei dem schlauchförmigen Ventilglied 25 um ein gummielastisches Formteil, das im weder von den Quetschelementen 28a, 28b noch von einem Fluid beaufschlagten Ausgangszustand allein aufgrund seiner inhärenten Stabilität eine sich ausgehend von den beiden Befestigungsflanschen 82 in Richtung zum Angriffsbereich der Quetschelemente 28a, 28b hin verjüngende Formgebung aufweist. Dies ist aus dem Längsschnitt der Figur 3 gut erkennbar.

Ein besonderer Vorteil des Quetschventils 1 resultiert aus einer Ausstattung mit zur elektrischen Signalabgabe ausgelegten Positionserfassungsmitteln 70, die eine berührungslose Erfassung mindestens einer Betriebsposition der Beaufschlagungsmittel 34 ermöglichen, die die Beaufschlagungsmittel im Rahmen der Beaufschlagungsbewegung einnehmen kann. Exemplarisch sind die Positionserfassungsmittel 70 derart ausgebildet und angeordnet, dass die beiden axialen Endlagen der Beaufschlagungsmittel 34 detektierbar sind, durch die zum einen die Offenstellung und zum anderen die Schließstellung des Quetschventils 1 vorgegeben wird.

Bevorzugt enthalten die Positionserfassungsmittel 70 an den Beaufschlagungsmitteln angeordnete und somit die Beaufschlagungsbewegung 38 unmittelbar mitmachende Betätigungsmittel 71, durch die darauf abgestimmte Sensormittel 73 betätigbar sind, die außerhalb des Arbeitsraumes am Ventilgehäuse 2 angeordnet sind. Die Betätigung erfolgt berührungslos.

Die Sensormittel 73 können weiterverwertbare elektrische Sensorsignale hervorrufen, beispielsweise um einer elektronischen Steuereinrichtung die aktuelle Position der Beaufschlagungsmittel 34 zu melden oder/und um optische und/oder akustische Meldemittel elektrisch zu aktivieren.

Bei den Betätigungsmitteln 71 handelt es sich insbesondere um eine Permanentmagneteinrichtung. Die Sensormittel 73 sind hierbei so ausgebildet, dass sie auf das von den Betätigungsmitteln 71 erzeugte Magnetfeld ansprechen. In diesem Zusammenhang ist es von Vorteil, wenn das Ventilgehäuse 2, insbesondere die umfangsseitige Ventilgehäusewand 5, aus einem nicht magnetischen und nicht magnetisierbaren Material besteht, sodass die magnetischen Feldlinien ungestört hindurchtreten können.

Exemplarisch bestehen die Sensormittel 73 aus einem permanentmagnetischen Ringmagnet 74, der koaxial zu der Ventilglied-Längsachse 32 an den Betätigungsmitteln 71 angeordnet ist.

Von Vorteil ist es, wenn die Betätigungsmittel 71 außerhalb der mit Fluid beaufschlagbaren Antriebskammer 58 an den Beaufschlagungsmitteln 34 angeordnet sind. Dadurch wird ein Kontakt mit dem Antriebsfluid vermieden, der Beschädigungen der Betätigungsmittel 71 zur Folge haben könnte.

In diesem Zusammenhang wird es beim Ausführungsbeispiel als zweckmäßig erachtet, die Betätigungsmittel 71 nicht an dem Antriebskolben 53 vorzusehen, sondern an dem Beaufschlagungsring 37. Für die Anbringung wird insbesondere eine Position an dem Beaufschlagungsring 37 gewählt, die zu keiner Zeit in den Bereich gelangt, der von dem Antriebskolben 53 bei der Betätigung des Quetschventils 1 überstrichen wird. Insbesondere sind die Betätigungsmittel 71 so angeordnet, dass sie sich unabhängig von der Betriebsposition der Beaufschlagungsmittel 38 stets auf der dem Antriebskolben 53 axial entgegengesetzten Seite der Quetschelemente 28a, 28b befinden.

Um die Montage der beim Ausführungsbeispiel ringförmigen Betätigungsmittel 71 zu erleichtern, ist der Beaufschlagungsring 37 bevorzugt in axialer Richtung zweigeteilt. Die Betätigungsmittel 71 sitzen axial zwischen einem ersten Ringelement 75 und einem zweiten Ringelement 76 des Beaufschlagungsringes 37: Das erste Ringelement 75 umfasst zweckmäßigerweise den Grundkörper 44 und die von diesem wegragenden Beaufschlagungsflügel 45a, 45b. An der von den Beaufschlagungsflügeln 45a, 45b wegweisenden Stirnseite verfügt der Grundkörper 44 über einen hülsenförmigen Lagerabschnitt 77 mit gegenüber dem Innendurchmesser des Arbeitsraumes 3 verringertem Außendurchmesser, auf den das zweite Ringelement 76 axial aufgesteckt ist. Auf spezielle Befestigungsmittel kann verzichtet werden, wenn sich die Federeinrichtung 65 seitens des Beaufschlagungsringes 37 an dem zweiten Ringelement 76 abstützt.

Die Sensormittel 73 sind radial neben dem Arbeitsraum 3 außerhal diesem in einem Bereich angeordnet, entlang dem sich die Beaufschlagungsmittel 34 beziehungsweise die daran angeordneten Betätigungsmittel 71 bei Ausführung der Beaufschlagungsbewegung verlagern. Die Sensormittel 73 liegen insbesondere radial neben der Bewegungsbahn der Betätigungsmittel 71.

Exemplarisch enthalten die Sensormittel 73 zwei jeweils auf eine bestimmte Betriebsposition der Beaufschlagungsmittel 34 ansprechende Positionssensoren 78, bei denen es sich beispielsweise um Hall-Sensoren oder um sogenannte Reed-Schalter handelt. Diese werden von dem Magnetfeld der Betätigungsmittel 71 zuverlässig erregt. Diese Zweizahl von Positionssensoren 78 ist allerdings nicht verbindlich. Soll nur eine Stellung erfasst werden, genügt ein einziger Positionssensor 78. Zur Erfassung von mehr als zwei Stellungen können auch entsprechend mehr als zwei Positionssensoren 78 verwendet werden.

Es besteht überdies die Möglichkeit, die Sensormittel 73 als Wegmesssystem auszuführen, mit denen sich eine stufenlose Positionserfassung der Beaufschlagungsmittel 34 durchführen lässt, entweder über den gesamten Verfahrweg der Beaufschlagungsmittel 34 oder über lediglich eine Teilstrecke desselben.

Die Sensormittel 73 rufen bei Aktivierung durch die Betätigungsmittel 71 ein oder mehrere elektrische(s) Sensorsignal(e) hervor, anhand dessen bzw. denen auf die aktuelle Axialposition der Beaufschlagungsmittel 34 und folglich auf den aktuellen Betriebszustand des Quetschventils 1 geschlossen werden kann.

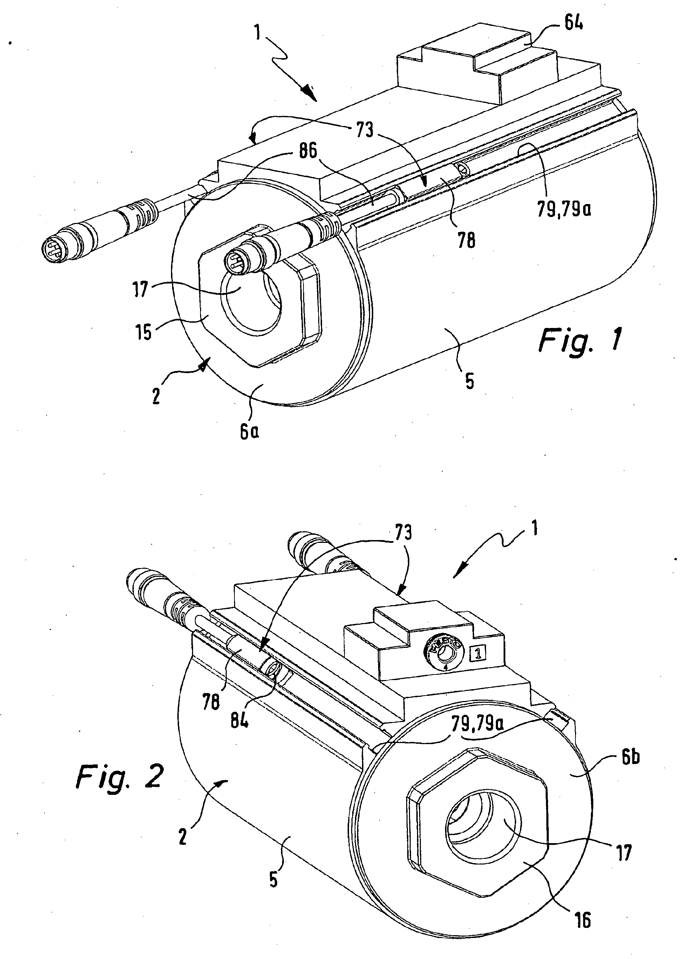

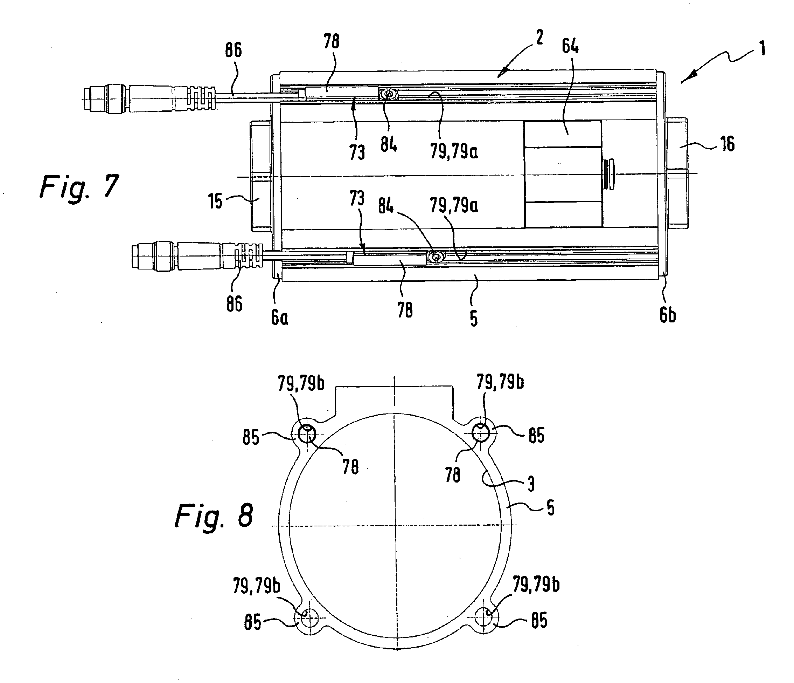

Zur Fixierung der Sensormittel 73 weist die umfangsseitige Ventilgehäusewand 5 zweckmäßigerweise eine oder mehrere sich in der Richtung der Beaufschlagungsbewegung 38 erstreckende Sensoraufnahmen 79 auf. Sind mehrere Sensoraufnahmen 79 vorhanden, liegt zweckmäßigerweise eine über den Umfang der umfangsseitigen Ventilgehäusewand 5 verteilte Anordnung vor. Bei dem Ausführungsbeispiel der Figuren 1 bis 7 sind zwei Sensoraufnahmen 79 vorhanden, bei dem Ausführungsbeispiel der Figur 8 insgesamt vier Sensoraufnahmen.

Bei dem erstgenannten Ausführungsbeispiel sind die Sensoraufnahmen 79 als im Querschnitt hinterschnittene Längsnuten 79a ausgeführt, die sich über die gesamte Länge der umfangsseitigen Ventilgehäusewand 5 erstrecken und zu deren beiden Stirnseiten ausmünden. Die Positionssensoren 78 können in diese Längsnuten 79a, je nach Ausführungsform, stirnseitig eingeschoben oder über die längsseitige, schlitzförmige Nutöffnung 83 hindurch eingesetzt werden. An den Positionssensoren 78 angeordnete Befestigungsmittel 84 gestatten eine lösbare Klemmfixierung an jeder beliebigen Längsposition innerhalb der Längsnut 79a.

Die Befestigungsmittel 84 enthalten beispielsweise in an sich bekannter Weise eine Klemmschraube, bei deren Drehung ein nicht weiter gezeigtes Klemmelement aktiviert wird, das dadurch mit der hinterschnittenen Querschnittskontur der Längsnut 79a verspannt wird.

Bei gelösten Befestigungsmitteln 84 kann jeder Positionssensor 78 in der Richtung der Beaufschlagungsbewegung 38 verstellt und nach Bedarf positioniert werden. Somit ergibt sich eine sehr variable Einstellmöglichkeit hinsichtlich den zu erfassenden Betriebspositionen.

Bei dem Ausführungsbeispiel der Figur 8 sind die Sensoraufnahmen 79 in Gestalt bohrungsartiger Kanäle 79b ausgeführt, die die umfangsseitige Ventilgehäusewand 5 axial durchziehen. Hier können die Sensormittel 73 komplett unsichtbar in der Ventilgehäusewand 5 untergebracht werden. Geeignete Positionssensoren 78 verfügen beispielsweise über einen komplementären Querschnitt und lassen sich reibungsbehaftet an der gewünschten Position fixieren.

Um trotz grundsätzlich geringer Wandstärke der umfangsseitigen Ventilgehäusewand 5 genügend Material zur Ausbildung der Sensoraufnahmen 79 zur Verfügung zu haben, ist die umfangsseitige Ventilgehäusewand 5 im Bereich der Sensoraufnahmen 79 zweckmäßigerweise mit je einer rippenartigen Erhebung 85 ausgestattet.

Besonders einfach lassen sich die Sensoraufnahmen 79 herstellen, wenn die umfangsseitige Ventilgehäusewand 5 von einem Profilrohr gebildet ist, das insbesondere durch Strangpressen hergestellt wurde. Das Einbringen der Sensoraufnahmen 79 erfordert hier keine mechanischen Nacharbeiten.

Bei dem Ausführungsbeispiel der Figur 8 können die bohrungsartigen Kanäle 79b auch zur stirnseitigen Befestigung der Abschlusswände 6a, 6b herangezogen werden. Die Kanäle 79b eignen sich beispielsweise zum Einschrauben selbstschneidender Befestigungsschrauben.

Die von den Sensormitteln 73 hervorgerufenen elektrischen Sensorsignale können beim Ausführungsbeispiel durch abgehende elektrische Kabel 86 zu einer nicht weiter abgebildeten elektronischen Auswerteeinrichtung übermittelt werden. Alternativ wäre allerdings auch eine drahtlose Signalübertragung denkbar.

Zusammenfassend lässt sich festhalten, dass bei dem Quetschventil 1 die für die Betätigung der Beaufschlagungsmittel 34 verantwortlichen Antriebsmittel 52, insbesondere der Antriebskolben 53, im Innern des Ventilgehäuses 2 untergebracht sind, was eine sehr schlanke Bauweise ermöglicht. Die Bewegungsrichtung 55 des Antriebskolbens 53 fällt mit der Achsrichtung des Ventilgliedes 25 zusammen, sodass er optimal in dem Arbeitsraum 3 untergebracht werden kann, in dem sich auch die Quetschelemente 28a, 28b befinden. Durch die berührungslose Positionserfassung der Beaufschlagungsmittel 34 ist eine bequeme und zuverlässige Betriebsüberwachung des Quetschventils 1 möglich, wobei von Vorteil ist, dass die Betätigungsmittel 71 nicht an den das Ventilglied 25 unmittelbar betätigenden Quetschelementen 28a, 28b angeordnet sind, sondern an den sich parallel zu dem Ventilglied 25 bewegenden Beaufschlagungsmitteln 34. Letzteres gilt übrigens auch dann, wenn die Antriebsmittel 52 abweichend vom Ausführungsbeispiel auf nicht-fluidischem Antriebsprinzip basieren und beispielsweise auf einem elektromotorischen oder elektromagnetischen Antriebsprinzip beruhen.

Die Quetschelemente 28a, 28b werden durch die Eigenspannung der als gummielastisches Formteil ausgebildeten Umfangswand 24 des schlauchförmigen Ventilgliedes 25 selbstständig in die Stellung "offen" radial in dem Führungsspalt 72 nach außen geschoben. Dabei folgen die Quetschelemente 28a, 28b den als schiefe Ebene ausgebildeten Beaufschlagungsflächen 46a, 46b an den Beaufschlagungsflügeln 45a, 45b. Diese Öffnungsbewegung der Quetschelemente 28a, 28b wird dadurch verstärkt, dass das gummielastische Ventilglied 25 in Richtung seiner Längsachse gestreckt ist, also mit Vorspannung installiert ist. Dies kann dadurch erzielt werden, dass das Innenmaß zwischen den sich axial gegenüberliegenden elastischen Befestigungsflanschen 82 kleiner ausgeführt ist als das entsprechende Außenmaß der darauf einwirkenden äußeren Stirnflächen der hier von den beiden Abstützhülsen 67, 68 gebildeten Abstützeinrichtung 43. Durch das axial gestreckte Ventilglied 25 ist bei jeder Stellung der Quetschelemente 28a, 28b ein Anliegen an den Beaufschlagungsflächen 46a, 46b sichergestellt.

## Patentansprüche

1. Quetschventil, mit einem sich zwischen zwei Anschlussstücken (15, 16) erstreckenden, eine flexible Umfangswand (24) aufweisenden schlauchförmigen Ventilglied (25), im Bereich dessen Außenumfanges mindestens ein zum mehr oder weniger starken Zusammenquetschen der Umfangswand (24) unter Ausführung einer Arbeitsbewegung (33) quer zu der Ventilglied-Längsachse (32) bewegbares Quetschelement (28a, 28b) angeordnet ist, mit im Bereich des Außenumfanges des Ventilgliedes (25) angeordneten Beaufschlagungsmitteln (34), die zu einer Beaufschlagungsbewegung (38) antreibbar sind, um durch Zusammenwirken mit dem mindestens einen Quetschelement (28a, 28b) dessen Arbeitsbewegung (33) hervorzurufen, mit einem Ventilgehäuse (2) mit einer umfangsseitigen Ventilgehäusewand (5), die einen die Beaufschlagungsmittel (34) enthaltenden Arbeitsraum (3) begrenzt, und mit für elektrische Signalabgabe ausgelegten Positionserfassungsmitteln (70) zur berührungslosen Erfassung mindestens einer im Rahmen der Beaufschlagungsbewegung (38) einnehmbaren Betriebsposition der Beaufschlagungsmittel (34), wobei die Positionserfassungsmittel (70) die Beaufschlagungsbewegung (38) mitmachende Betätigungsmittel (71) sowie außerhalb des Arbeitsraumes (3) ventilgehäusefest angeordnete, berührungslos auf die Betätigungsmittel (71) ansprechende Sensormittel (73) enthalten, **dadurch gekennzeichnet, dass** die Ventilgehäusewand (5) das Ventilglied (25) radial außen umschließt und dass die Beaufschlagungsbewegung (38) der Beaufschlagungsmittel (34) längs des Ventilgliedes (25) orientiert ist, wobei die Betätigungsmittel (71) an den Beaufschlagungsmitteln (34) angeordnet sind.

2. Quetschventil nach Anspruch 1, **dadurch gekennzeichnet, dass** die Betätigungsmittel (71) aus einer Permanentmagneteinrichtung bestehen, die zweckmäßigerweise mindestens einen das Ventilglied (25) koaxial umschließenden Ringmagnet (74) enthält.

3. Quetschventil nach Anspruch 1 oder 2, **dadurch gekennzeichnet, dass** die Sensormittel (73) mindestens einen auf eine bestimmte Betriebsposition ansprechenden Positionssensor (78) enthalten, insbesondere ein Hall-Sensor oder ein Reed-Schalter.

4. Quetschventil nach einem der Ansprüche 1 bis 3, **dadurch gekennzeichnet, dass** die Sensormittel (73) längsseits neben
dem Arbeitsraum (3) in einem Bereich angeordnet sind, entlang dem sich die Beaufschlagungsmittel (34) bei ihrer Beaufschlagungsbewegung (38) verlagern.

5. Quetschventil nach einem der Ansprüche 1 bis 4, **dadurch gekennzeichnet, dass** die Sensormittel (73) in der Richtung der Beaufschlagungsbewegung (38) einstellbar am Ventilgehäuse (2) angeordnet sind.

6. Quetschventil nach einem der Ansprüche 1 bis 5, **dadurch gekennzeichnet, dass** die umfangsseitige Ventilgehäusewand (5) mindestens eine sich in der Richtung der Beaufschlagungsbewegung (38) erstreckende Sensoraufnahme (79) zur Fixierung der Sensormittel (73) aufweist.

7. Quetschventil nach Anspruch 6, **dadurch gekennzeichnet, dass** mindestens eine Sensoraufnahme (79) als hinterschnittene Längsnut (79a) und/oder als bohrungsartiger Kanal (79b) und/oder in einer am Außenumfang der umfangsseitigen Ventilgehäusewand (5) angeordneten rippenartigen Erhebung (85) ausgebildet ist.

8. Quetschventil nach einem der Ansprüche 1 bis 7, **dadurch gekennzeichnet, dass** zwei sich bezüglich der Ventilglied-Längsachse (32) diametral gegenüberliegende, sich bei Ausführung der Arbeitsbewegung (33) aufeinander zu oder voneinander weg bewegende Quetschelemente (28a, 28b) im Bereich des Außenumfanges des Ventilgliedes (25) angeordnet sind.

9. Quetschventil nach einem der Ansprüche 1 bis 8, **dadurch gekennzeichnet, dass** die Beaufschlagungsmittel (34) ring- oder hülsenförmig ausgebildet sind und das schlauchförmige Ventilglied (25) im Innern des Arbeitsraumes (3) koaxial umschließen.

10. Quetschventil nach einem der Ansprüche 1 bis 9, **dadurch gekennzeichnet, dass** die Beaufschlagungsmittel (34) einen das Ventilglied (25) koaxial umschließenden Beaufschlagungsring (37) enthalten, der in dem Arbeitsraum (3) angeordnet ist und der mindestens eine zum Zusammenwirken mit dem mindestens einen Quetschelement (28a, 28b) vorgesehene Beaufschlagungsfläche (46a, 46b) aufweist.

11. Quetschventil nach Anspruch 10, **dadurch gekennzeichnet, dass** die Betätigungsmittel (71) der Positionserfassungsmittel (70) an dem Beaufschlagungsring (37) angeordnet sind.

12. Quetschventil nach einem der Ansprüche 1 bis 11, **dadurch gekennzeichnet, dass** es mit Antriebsmitteln (52) zum Hervorrufen einer für die Beaufschlagungsbewegung (38) verantwortlichen Antriebskraft ausgestattet ist, wobei die Antriebsmittel (52) zweckmäßigerweise für durch Fluidkraft gesteuerte Erzeugung der Antriebskraft ausgebildet sind und wobei die Antriebsmittel (52) eine axial benachbart zu den Beaufschlagungsmitteln (34) in dem Arbeitsraum (3) ausgebildete, zur Fluidbeaufschlagung geeignete und das Ventilglied (25) koaxial umschließende ringförmige Antriebskammer (58) sowie mindestens einen mit der Antriebskammer (58) kommunizierenden, eine gesteuerte Fluidzufuhr und/oder Fluidabfuhr bezüglich der Antriebskammer (58) ermöglichenden fluidischen Steuerkanal (62) enthalten, wobei in dem Arbeitsraum (3) ein die Antriebskammer (58) axial begrenzender, durch gesteuerte Fluidbeaufschlagung der Antriebskammer (58) axial beweglicher, ringförmiger Antriebskolben (53) angeordnet ist, der das Ventilglied (25) koaxial umschließt und dessen Bewegung die Beaufschlagungsbewegung (38) der Beaufschlagungsmitteln (34) hervorruft.

13. Quetschventil nach Anspruch 12, **dadurch gekennzeichnet, dass** die Beaufschlagungsmittel (34) einen mit den Quetschelementen (28a, 28b) zusammenwirkenden Beaufschlagungsring (37) enthalten, der ein von dem Antriebskolben (53) unabhängiges, separates Bauteil ist, das sich insbesondere ohne feste Verbindung axial an dem Antriebskolben (53) abstützt.

14. Quetschventil nach Anspruch 12 oder 13, **dadurch gekennzeichnet, dass** die Betätigungsmittel (34) der Positionserfassungsmittel (70) außerhalb der vom Antriebskolben (53) begrenzten, mit Fluid beaufschlagbaren Antriebskammer (58) an den Beaufschlagungsmitteln (34) angeordnet sind.

## Claims

1. Pinch valve, comprising a tubular valve member (25) extending between two connecting pieces (15, 16) and having a flexible circumferential wall (24), in the region of the outer circumference of which valve member (25) at least one pinch element (28a, 28b) movable perpendicular to the valve member longitudinal axis (32) for the more or less strong pinching of the circumferential wall (24) while performing an operating movement (33) is located, further comprising impingement means (34) located in the region of the outer circumference of the valve member (25), which can be driven to perform a impingement movement (38) in order to cause the operating movement of the at least one pinch element (28a, 28b) in cooperation therewith, further comprising a valve housing (2) with a circumferential valve housing wall (5), which bounds an operating chamber (3) containing the impingement means (34), and further comprising position detecting means (70) designed for outputting electric signals for the non-contact detection of at least one operating position the impingement means (34) can adopt within the impingement movement (38), wherein the position detecting means (70) comprise actuating means (71) joining the impingement movement (38) and sensor means (73), which are mounted on the valve housing outside the operating chamber (3) and which respond to the actuating means (71) in a non-contact manner, **characterised in that** the valve housing wall (5) radially encloses the valve member (25) on the outside, and **in that** the impingement movement (38) of the impingement means (34) is oriented along the valve member (25), the actuating means (71) being located on the impingement means (34).

2. Pinch valve according to claim 1, **characterised in that** the actuating means (71) consist of a permanent magnet device which expediently comprises at least one annular magnet (74) coaxially enclosing the valve member (25).

3. Pinch valve according to claim 1 or 2, **characterised in that** the sensor means (73) comprise at least one position sensor (78), in particular a Hall sensor or a reed switch, which responds to a defined operating position.

4. Pinch valve according to any of claims 1 to 3, **characterised in that** the sensor means (73) are located along the operating chamber (3) in a region along which the impingement means (34) are displaced during their impingement movement (38).

5. Pinch valve according to any of claims 1 to 4, **characterised in that** the sensor means (73) are arranged on the valve housing (2) for adjustment in the direction of the impingement movement (38).

6. Pinch valve according to any of claims 1 to 5, **characterised in that** the circumferential valve housing wall (5) comprises at least one sensor receptacle (79) extending in the direction of the impingement movement (38) for locating the sensor means (73).

7. Pinch valve according to claim 6, **characterised in that** the at least one sensor receptacle (79) is designed as an undercut longitudinal groove (79a) and/or as a boretype passage (79b) and/or a rib-type raised area (85) on the outer circumference of the circumferential valve housing wall (5).

8. Pinch valve according to any of claims 1 to 7, **characterised in that** two pinch elements (28a, 28b), which are placed diametrically opposite one another relative to the valve member longitudinal axis (32) and which move towards one another or away from one another when executing the operating movement (33), are located in the region of the outer circumference of the valve member (25).

9. Pinch valve according to any of claims 1 to 8, **characterised in that** the impingement means (34) are designed to be annular or sleeve-shaped and coaxially enclose the tubular valve member (25) in the interior of the operating chamber (3).

10. Pinch valve according to any of claims 1 to 9, **characterised in that** the impingement means (34) comprise a impingement ring (37), which coaxially encloses the valve member (25), which is located in the operating chamber (3) and which has at least one impingement surface (46a, 46b) designed for acting together with the at least one pinch element (28a, 28b).

11. Pinch valve according to claim 10, **characterised in that** the actuating means (71) of the position detecting means (70) are located on the impingement ring (37).

12. Pinch valve according to any of claims 1 to 11, **characterised in that** it is provided with drive means (52) responsible for generating a driving force for the impingement movement (38), wherein the drive means (52) are expediently designed for a fluid power-controlled generation of the driving force and wherein the drive means (52) comprise an annular drive chamber (58) formed axially adjacent to the impingement means (34) in the operating chamber (3) and coaxially enclosing the valve member (25), as well as at least one fluidic control passage (62) communicating with the drive chamber (58) and facilitating a controlled fluid supply to and/or fluid discharge relative to the drive chamber (58), wherein an annular drive piston (51), which axially bounds the drive chamber (58), is axially movable by the controlled application of fluid pressure to the drive chamber (58) and coaxially encloses the valve member (25) and by its movement causes the impingement movement (38) of the impingement means (34), is located in the operating chamber (3).

13. Pinch valve according to claim 12, **characterised in that** the impingement means (34) comprise a impingement ring (37), which acts together with the pinch elements (28a, 28b) and which is a component independent of the drive piston (53), being in particular axially supported on the drive piston (53) without any permanent connection.

14. Pinch valve according to claim 12 or 13, **characterised in that** the actuating means (34) of the position sensing means (70) are located on the impingement means (34) outside the operating chamber (58), which is bounded by the drive piston (53) and which can be pressurised by fluid.

## Revendications

1. Soupape à pincement avec un organe de soupape (25) en forme de tuyau, présentant une paroi périphérique flexible (24), s'étendant entre deux pièces de raccordement (15, 16), dans la zone de la périphérie extérieure duquel au moins un écrasement plus ou moins fort de la paroi périphérique (24) est agencé en réalisant un mouvement de travail (33) transversalement à l'axe longitudinal d'organe de soupape (32) de l'élément de pincement (28a, 28b) mobile, avec des moyens d'alimentation (34) agencés dans la zone de la périphérie extérieure de l'organe de soupape (25) qui sont entraînables pour un mouvement d'alimentation (38) afin de susciter par coaction avec l'au moins un élément de pincement (28a, 28b) leur mouvement de travail (33), avec un boîtier de soupape (2) présentant une paroi de boîtier de soupape (5) côté périphérie qui délimite un espace de travail (3) contenant les moyens d'alimentation (34), et avec des moyens de détection de position (70) conçus pour une émission de signal électrique pour la détection sans contact au moins d'une position de fonctionnement pouvant être occupée dans le cadre du mouvement d'alimentation (38) des moyens d'alimentation (34), les moyens de détection de position (70) contenant des moyens d'actionnement (71) participant au mouvement d'alimentation (38) ainsi que des moyens de capteur (73) sensibles sans contact aux moyens d'actionnement (71), agencés en dehors de l'espace de travail (3) fixement au boîtier de soupape, **caractérisée en ce que** la paroi de boîtier de soupape (5) entoure radialement à l'extérieur l'organe de soupape (25) et **en ce que** le mouvement d'alimentation (38) des moyens d'alimentation (34) est orienté le long de l'organe de soupape (25), les moyens d'actionnement (71) étant agencés sur les moyens d'alimentation (34).

2. Soupape à pincement selon la revendication 1, **caractérisée en ce que** les moyens d'actionnement (71) se composent d'un dispositif à aimant permanent qui contient de manière adaptée au moins un aimant annulaire (74) entourant coaxialement l'organe de soupape (25).

3. Soupape à pincement selon la revendication 1 ou 2, **caractérisée en ce que** les moyens de capteur (73) contiennent au moins un capteur de position (78) sensible à une position de fonctionnement déterminée, en particulier un capteur à effet Hall ou un commutateur Reed.

4. Soupape à pincement selon l'une quelconque des revendications 1 à 3, **caractérisée en ce que** les moyens de capteur (73) sont agencés côté longueur à côté de l'espace de travail (3) dans une zone, le long de laquelle les moyens d'alimentation (34) se déplacent pour leur mouvement d'alimentation (38).

5. Soupape à pincement selon l'une quelconque des revendications 1 à 4, **caractérisée en ce que** les moyens de capteur (73) sont agencés de manière réglable dans la direction du mouvement d'alimentation (38) sur le boîtier de soupape (2).

6. Soupape à pincement selon l'une quelconque des revendications 1 à 5, **caractérisée en ce que** la paroi de boîtier de soupape (5) côté périphérie présente au moins un logement de capteur (79) s'étendant dans la direction du mouvement d'alimentation (38) pour la fixation des moyens de capteur (73).

7. Soupape à pincement selon la revendication 6, **caractérisée en ce qu'**au moins un logement de capteur (79) est réalisé comme une rainure longitudinale (79a) contre-dépouillée et/ou comme un canal de type perçage (79b) et/ou dans une élévation (85) de type nervure agencée sur la périphérie extérieure de la paroi de boîtier de soupape (5) côté périphérie.

8. Soupape à pincement selon l'une quelconque des revendications 1 à 7, **caractérisée en ce que** deux éléments de pincement (28a, 28b) se déplaçant vers ou loin l'un de l'autre pour la réalisation du mouvement de travail (33), opposés diamétralement par rapport à l'axe longitudinal d'organe de soupape (32) sont agencés dans la zone de la périphérie extérieure de l'organe de soupape (25).

9. Soupape à pincement selon l'une quelconque des revendications 1 à 8, **caractérisée en ce que** les moyens d'alimentation (34) sont réalisés en forme d'anneau ou de douille et entourent coaxialement l'organe de soupape (25) en forme de tuyau à l'intérieur de l'espace de travail (3).

10. Soupape à pincement selon l'une quelconque des revendications 1 à 9, **caractérisée en ce que** les moyens d'alimentation (34) contiennent un anneau d'alimentation (37) entourant coaxialement l'organe de soupape (25), lequel anneau est agencé dans l'espace de travail (3) et présente au moins une surface d'alimentation (46a, 46b) prévue pour la coaction avec l'au moins un élément de pincement (28a, 28b).

11. Soupape à pincement selon la revendication 10, **caractérisée en ce que** les moyens d'actionnement (71) des moyens de détection de position (70) sont agencés sur l'anneau d'alimentation (37).

12. Soupape à pincement selon l'une quelconque des revendications 1 à 11, **caractérisée en ce qu'**elle est équipée de moyens d'entraînement (52) pour susciter une force d'entraînement responsable du mouvement d'alimentation (38), les moyens d'entraînement (52) étant réalisés de manière appropriée pour la génération commandée par la force fluidique de la force d'entraînement et les moyens d'entraînement (52) contenant une chambre d'entraînement (58) annulaire réalisée de manière axialement contiguë aux moyens d'alimentation (34) dans l'espace de travail (3), appropriée à l'alimentation fluidique et entourant coaxialement l'organe de soupape (25) ainsi qu'au moins un canal de commande (62) fluidique communiquant avec la chambre d'entraînement (58), permettant une amenée de fluide et/ou une évacuation de fluide commandée par rapport à la chambre d'entraînement (58), dans l'espace de travail (3) étant agencé un piston d'entraînement (53) annulaire, mobile axialement par alimentation fluidique commandée de la chambre d'entraînement (58), délimitant axialement la chambre d'entraînement (58), lequel piston entoure coaxialement l'organe de soupape (25) et dont le mouvement suscite le mouvement d'alimentation (38) des moyens d'alimentation (34).

13. Soupape à pincement selon la revendication 12, **caractérisée en ce que** les moyens d'alimentation (34) contiennent un anneau d'alimentation (37) coagissant avec les éléments de pincement (28a, 28b), qui est un composant séparé indépendant du piston d'entraînement (53), et s'appuie en particulier sans liaison fixe axialement contre le piston d'entraînement (53).

14. Soupape à pincement selon la revendication 12 ou 13, **caractérisée en ce que** les moyens d'actionnement (34) des moyens de détection de position (70) sont agencés sur les moyens d'alimentation (34) en dehors de la chambre d'entraînement (58) pouvant être alimentée en fluide, délimitée par le piston d'entraînement (53).
